# EUROPEAN PATENT SPECIFICATION

(11) **EP 2 112 904 B1**
(45) Date of publication and mention of the grant of the patent: **01.04.2015**
(21) Application number: 08710233.1
(22) Date of filing: 21.02.2008
(51) Int. Cl.: A61B 5/05, A61B 5/029, A61B 5/083

(54) **METHOD AND SYSTEM FOR ESTIMATING EXERCISE CAPACITY**
VERFAHREN UND SYSTEM ZUR SCHÄTZUNG DER KÖRPERLICHEN BEWEGUNGSKAPAZITÄT
PROCÉDÉ ET SYSTÈME PERMETTANT D'ESTIMER UNE APTITUDE À L'EXERCICE

(30) Priority: 23.02.2007 US 902853 P; 13.08.2007 US 889395
(43) Date of publication of application: 04.11.2009
(73) Proprietor: Cheetah Medical, Inc., Wilmington, DE 19808 (US)
(72) Inventor: KEREN, Hanan, 44235 Kfar-Saba (IL); BURKHOFF, Daniel, West Harrison, New York 10605 (US); AVIDOR, Yoav, 62304 Tel-Aviv (IL); SQUARA, Pierre, F-95880 Enghien-Les-Bains (FR)
(74) Representative: Vossius & Partner
(86) International application number: PCT/IL2008/000233
(87) International publication number: WO 2008/102362

(56) References cited:
- WO-A-2006/087696
- US-A- 5 158 093
- US-A1- 2005 124 901
- US-A1- 2005 192 488
- LELE SUHAS S ET AL: "Exercise capacity in hypertrophic cardiomyopathy: Role of stroke volume limitation, heart rate, and diastolic filling characteristics" CIRCULATION, vol. 92, no. 10, 1995, pages 2886-2894, XP002487808 ISSN: 0009-7322
- RAZA S B ET AL: "FILTERING RESPIRATION AND LOW-FREQUENCY MOVEMENT ARTEFACTS FROM THE CARDIOGENIC ELECTRICAL IMPEDANCE SIGNAL" MEDICAL AND BIOLOGICAL ENGINEERING AND COMPUTING, SPRINGER, HEILDELBERG, DE, vol. 30, no. 5, 1 September 1992 (1992-09-01), pages 556-561, XP000323425 ISSN: 0140-0118
- MIYAMOTO Y ET AL: "CARDIO RESPIRATORY DYNAMICS DURING SINUSOIDAL AND IMPULSE EXERCISE IN MAN" JAPANESE JOURNAL OF PHYSIOLOGY, vol. 33, no. 6, 1983, pages 971-986, XP008094022 ISSN: 0021-521X
- NEWMAN DAVID G ET AL: "The non-invasive assessment of stroke volume and cardiac output by impedance cardiography: A review" AVIATION SPACE AND ENVIRONMENTAL MEDICINE, vol. 70, no. 8, August 1999 (1999-08), pages 780-789, XP008093991 ISSN: 0095-6562 cited in the application
- MYERS ET AL: "Cardiac Output and Cardiopulmonary Responses to Exercise in Heart Failure: Application of a New Bio-Reactance Device" JOURNAL OF CARDIAL FAILURE, CHURCHILL LIVINGSTONE, NAPERVILLE, IL, US, vol. 13, no. 8, 6 October 2007 (2007-10-06), pages 629-636, XP022287147 ISSN: 1071-9164
- YAMAMOTO Y ET AL: "DESIGN AND IMPLEMENTATION OF A DIGITAL FILTER FOR BEAT-BY-BEAT IMPEDANCE CARDIOGRAPHY", IEEE TRANSACTIONS ON BIOMEDICAL ENGINEERING, IEEE SERVICE CENTER, PISCATAWAY, NJ, USA, vol. 35, no. 12, 1 December 1988 (1988-12-01), pages 1086-1090, XP000068625, ISSN: 0018-9294, DOI: 10.1109/10.8694

## Description

### FIELD AND BACKGROUND OF THE INVENTION

The present invention, in some embodiments thereof, relates to medical application and, more particularly, but not exclusively, to a method and system for estimating exercise capacity.

Congestive Heart Failure (CHF) is a chronic inability of the heart to maintain an adequate output of blood from one or both ventricles of the heart to meet the metabolic demands of the tissues. In patients diagnosed with CHF, sudden cardiac death occurs at 6 to 9 times the rate of the general population.

In CHF subjects having a markedly weakened left ventricle and not markedly weakened right ventricle, blood continues to be pumped into the lungs but is not pumped adequately out of the lungs. As the volume of blood in the lungs increases, the pulmonary vessels enlarge, pulmonary venous congestion develops, and, once the pulmonary capillary pressure rises above a critical point, fluid begins to filter out of the capillaries into the interstitial spaces and alveoli, resulting in pulmonary edema. Pulmonary edema may lead to pleural and abdominal effusion.

In CHF subjects having abnormality in the right heart or the pulmonary arteries, the ability of the heart to move blood forward is limited and congestion occurs behind the right heart. Such congestion causes pleural effusion and/or buildup of fluid in the abdomen.

Numerous techniques for predicting and determining the condition of CHF subjects are known. One such technique is known as exercise capacity test. In exercise capacity test, the respiratory and/or circulatory response of the subject to exercise is analyzed to stratify risk and assess therapy. Analysis of expired gas during exercise is commonly known as cardiopulmonary exercise testing (CPX) or metabolic exercise.

CPX typically includes the calculation or estimation of several measures. Oxygen uptake, also known as VO₂, is the rate of oxygen consumption by a patient during an exercise test. Peak VO₂ is considered as a measure of a patient's aerobic exercise capacity, and has traditionally been considered a surrogate for maximal cardiac output. Carbon dioxide production rate, also known as VCO₂, is the rate of carbon dioxide production by a patient during exercise. VCO₂ relative to VO₂ is influenced by which substrate is metabolized (fat vs. carbohydrate) and whether anaerobic processes and lactic acid production occur. Known in the art are methods of calculating VO₂ and VCO₂ by numerical integration of the product of expiratory airflow with O₂ and CO₂ concentrations.

Attempts have been made to developed techniques for estimating cardiac output during exercise. Generally, direct measurement of cardiac output by thermodilution is considered the "gold standard." Yet, this technique is cumbersome, invasive, and carries an added expense and degree of risk. Several non invasive techniques are described in Leslien et al., "Non-invasive measurement of cardiac output in patients with chronic heart falure,", Blood Press Monit 2004, 9:277-280; Engoren et al., "Comparison of cardiac output determined by bioimpedance, thermodilution, and the Fick method,", Am J Crit Care 2005, 14:40-45; and Newman et al., "The non-invasive assessment of stroke volume and cardiac output by impedance cardiography: a review," Aviat Space Environ Med, 1999, 70:780-789.

Lele et al., "Exercise Capacity in Hypertrophic Cardiomyopathy", Circulation vol. 92, pp. 2886-2894, discloses determining exercise capacity from cardiac output.

Raza et al., "Filtering respiration and low-frequency movement artefacts from the cardiogenic electrical impedance signal", Medical and Biological Engineering and Computing vol. 30, pp. 556-561 discloses a low-pass filter adaptable to physiological data based on impedance cardiography.

Yamamoto et al., "Design and Implementation of a Digital Filter for Beat-by-Beat Impedance Cardiography", IEEE Trans. Biomed. Eng. vol. 35 pp. 1086-1090, discloses a dynamically variable band pass filter being applied to impedance cardiography signals.

### SUMMARY OF THE INVENTION

According an aspect of the present invention there is provided a method of estimating exercise capacity of a subject using output radiofrequency signals transmitted to the subject during exercise and input radiofrequency signals received from the subject during exercise. The method comprises: processing said input radiofrequency signal, wherein said processing comprises determining a phase shift of the input radiofrequency signals relative to the output radiofrequency signals, to provide a processed input signal which comprises said phase shift; filtering the processed signal using a dynamically variable band pass filter charachterized by a frequency band which is dynamically adapted in response to a change in a physiological condition of the subject, thereby providing a filtered signal; calculating cardiac output based on the phase shift; and using the cardiac output for estimating the exercise capacity of the subject.

According to some embodiments of the invention, the method further comprising obtaining at least one CPX measure pertaining to a cardiopulmonary exercise testing, and combining the cardiac output with the at least one CPX measure to estimate the exercise capacity of the subject.

According to some embodiments of the invention, the method further comprises reducing or eliminating amplitude modulation of the input radiofrequency signals so as to provide input radiofrequency signals of substantially constant envelope.

According to some embodiments of the invention the method further comprises mixing the output radiofrequency signals and the input radiofrequency signals so as to provide a mixed radiofrequency signal, and filtering out a portion of the mixed radiofrequency signal so as to substantially increase a signal-to-noise ratio of a remaining portion of the mixed radiofrequency signal.

According an aspect of the present invention there is provided apparatus for estimating exercise capacity of a subject using output radiofrequency signals transmitted to the subject during exercise and input radiofrequency signals received from the subject during exercise. The apparatus comprises: a phase shift determinator configured for determining a phase shift of the input radiofrequency signals relative to the output radiofrequency signals, to provide a processed siganl which comprises the phase shift; a filtering unit configured for filtering said processed signal using a dynamically variable band pass filter characterized by a frequency band which is dynamically adapted in response to a change in a physiological condition of the subject, to thereby provide a filtered signal; and a cardiac output calculator configured for calculating cardiac output based on the phase shift; and an exercise capacity estimator configured for using the cardiac output for estimating the exercise capacity of the subject.

According to some embodiments of the invention the exercise capacity estimator is configured for combining the cardiac output with at least one CPX measure to estimate the exercise capacity of the subject.

According to an aspect of the present invention there is provided a system for estimating exercise capacity of a subject. The system comprises : a radiofrequency generator for generating output radiofrequency signals; a plurality of electrodes designed for transmitting the output radiofrequency signals to the subject and for sensing input radiofrequency signals from the subject; and an apparatus for estimating exercise capacity of a subject.

According to some embodiments of the invention, the system further comprises a cardiopulmonary exercise testing system configured to provide at least one CPX measure, wherein the exercise capacity estimator is configured for combining the cardiac output with at least one CPX measure to estimate the exercise capacity of the subject.

According to some embodiments of the invention, the apparatus further comprises an envelope elimination unit designed and configured for reducing or eliminating amplitude modulation of the input radiofrequency signals so as to provide input radiofrequency signals of substantially constant envelope.

According to some embodiments of the invention the apparatus further comprises a mixer configured for mixing the output radiofrequency signals and the input radiofrequency signals, to provide a mixed radiofrequency signal; and a radiofrequency filter for filtering out a portion of the mixed radiofrequency signal so as to substantially increase a signal-to-noise ratio of a remaining portion of the mixed radiofrequency signal.

According to some embodiments of the invention the cardiac output is calculated using a linear relationship between the phase shift and the cardiac output.

According to some embodiments of the invention the dynamically variable filter is adapted in response to a change in a physiological condition of the subject.

According to some embodiments of the invention the physiological condition is a heart rate of the subject.

According to some embodiments of the invention a lower frequency bound characterizing the filter is about 0.9*(HR/60) Hz at all times, wherein the HR is the heart rate in units of beats per minute.

According to some embodiments of the invention an upper frequency bound characterizing the filter is about 6 + 1.5*[(HR/60) - 1] Hz at all times, wherein the HR is the heart rate in units of beats per minute.

According to some embodiments of the invention the CPX measure(s) comprises ratio of ventilation efficiency to carbon dioxide production rate.

According to some embodiments of the invention the CPX measure(s) comprises oxygen uptake efficiency slope.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a flowchart diagram of a method suitable for estimating exercise capacity of a subject according to various exemplary embodiments of the present invention;
FIG. 2 is a flowchart diagram of a more detailed method suitable for estimating exercise capacity of a subject according to various exemplary embodiments of the present invention;
FIGs. 3a-b show a representative example of dynamically varying frequency bounds, employed according to embodiments of the present invention;
FIG. 3c show a representative example of a dynamically varying frequency band, employed according to embodiments of the present invention;
FIG. 4 is a schematic illustration of apparatus for estimating exercise capacity of a subject, according to various exemplary embodiments of the present invention;
FIG. 5 is a schematic illustration of a signal processing unit, according to various exemplary embodiments of the present invention;
FIG. 6 is a block diagram of electronic circuitry, according to various exemplary embodiments of the present invention;
FIG. 7 is a schematic illustration of a system for estimating exercise capacity of a subject;
FIG. 8 is a graph showing mean values for oxygen uptake during exercise superimposed with mean changes in cardiac output in a typical patient with CHF as obtained according to some embodiments of the present invention;
FIG. 9 is a graph showing the relationship between cardiac output and oxygen uptake at rest and peak exercise according to some embodiments of the present invention;
FIG. 10 is a graph showing relationships between peak cardiac index and peak VO₂ in CHF patients tested according to some embodiments of the present invention, and patients with CAD tested using a direct Fick method;
FIG. 11 is a graph showing the association between resting and peak exercise cardiac output and oxygen uptake according to some embodiments of the present invention, superimposed with other studies;
FIG. 12 is a graph showing the relationship between cardiac output as determined according to some embodiments of the present invention and age;
FIG. 13 is a graph showing the relationship between maximal cardiac output as calculated according to some embodiments of the present invention and the VE/VCO₂ slope; and
FIG. 14 is a graph showing the relationship between maximal cardiac output as calculated according to some embodiments of the present invention and the OUES;.

### DESCRIPTION OF SPECIFIC EMBODIMENTS OF THE INVENTION

The present invention, in some embodiments thereof, relates to medical application and, more particularly, but not exclusively, to a method and system for estimating exercise capacity.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

Computer programs implementing the method according to embodiments of the present invention can commonly be distributed to users on a distribution medium such as, but not limited to, a floppy disk, CD-ROM and flash memory cards. From the distribution medium, the computer programs can be copied to a hard disk or a similar intermediate storage medium. The computer programs can be run by loading the computer instructions either from their distribution medium or their intermediate storage medium into the execution memory of the computer, configuring the computer to act in accordance with the method of this invention. All these operations are well-known to those skilled in the art of computer systems.

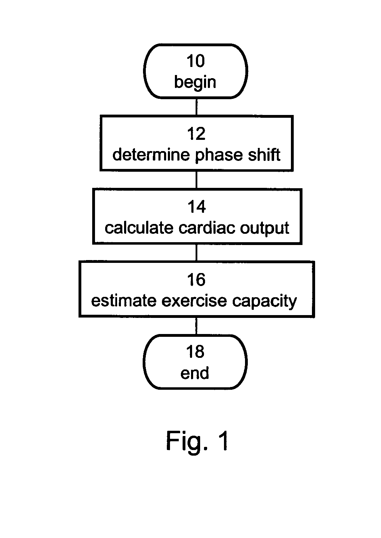

Referring now to the drawings, Figure 1 is a flowchart diagram of a method suitable for estimating exercise capacity of a subject according to various exemplary embodiments of the present invention.

It is to be understood that, unless otherwise defined, the method steps described hereinbelow can be executed either contemporaneously or sequentially in many combinations or orders of execution. Specifically, the ordering of the flowcharts diagrams is not to be considered as limiting. For example, two or more method steps, appearing in the following description or in the flowchart diagrams in a particular order, can be executed in a different order (*e.g*., a reverse order) or substantially contemporaneously. Additionally, several method steps described below are optional and may not be executed.

The method is particularly useful for estimating the exercise capacity from output radiofrequency signals transmitted to the subject during exercise and input radiofrequency signals received from the subject during exercise.

The method begins at step **10** and continues to step **12** in which a phase shift of the input radiofrequency signals relative to the output radiofrequency signals is determined. The method continues to step **14** in which a cardiac output is calculated, based on the phase shift. The method continues to step **16** in which the cardiac output is used for estimating the exercise capacity of the subject.

The method ends at step **18.**

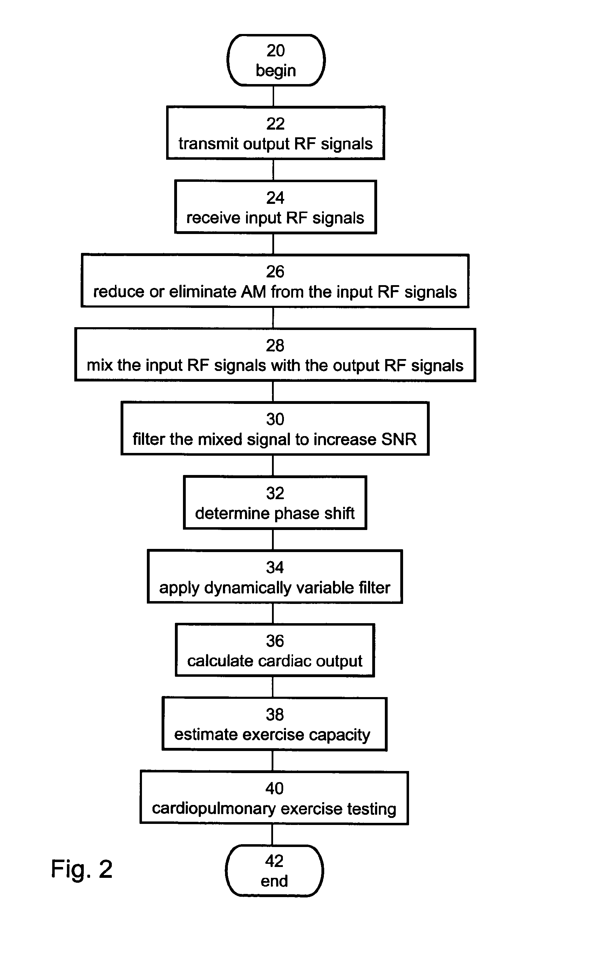

A more detailed method for estimating exercise capacity of a subject according to some embodiments of the present invention is illustrated in the flowchart diagram of Figure 2.

The method begins at step **20** and optionally continues to step **22** in which output radiofrequency signals are transmitted to the subject during exercise, and step **24** in which input radiofrequency signals are received from the subject during exercise. The output radiofrequency signals can be generated by a radiofrequency generator which generates a periodic high frequency current output in response to a periodic control input signal. The current output can be transmitted to the subject via an arrangement of electrodes for carrying current output from the radiofrequency generator as known in the art. The electrodes can be connected to locations of the body of the subject, *e.g*., above and below the heart. Since the transmission and reception of signals is done during exercise, the electrodes are optionally and preferably placed on the subject's back so as not to interfere with upper body motion.

Current, generated by the radiofrequency generator, flows across the thorax and causes a voltage drop due to the impedance of the body. The input radiofrequency signals are typically, but not obligatorily, relate to the hemodynamic reactance of an organ of the subject.

As used herein, "hemodynamic reactance" refers to the imaginary part of the impedance. Techniques for extracting the imaginary part from the total impedance are known in the art. Typically, such extraction is performed at hardware level but the use of algorithm at a software level is not excluded from the scope of the present invention.

According to some embodiments of the present invention the method continues to step **26** in which amplitude modulation of the input radiofrequency signals is reduces or, more preferably, eliminated. Optionally and preferably the phase modulation of the signals is maintained. The input radiofrequency signals typically carry a substantial amount of AM noise, which can be described, without limitation as a signal *v*(*t*)*cos*(*ωt*+*ϕ*(*t*)), which contains both phase and amplitude modulation. According to some embodiments the method generates signals having a substantial constant envelope, *e.g., v*₀cos(*ωt+ϕ*(*t*))*,* where *v*₀ is substantially a constant. The obtained signals thus represent the phase (or frequency) modulation of the input radiofrequency signal. The reduction or elimination of the amplitude modulation can be achieved, for example, using a limiter amplifier which amplifies the radiofrequency signals and limits their amplitude such that the amplitude modulation is removed.

In some embodiments, the method proceeds to step **28** in which the output radiofrequency signals are mixed with the input radiofrequency signals so as to provide a mixed radiofrequency signal. According to a preferred embodiment of the present invention, the mixed radiofrequency signal is composed of a plurality of radiofrequency signals, which may be, in one embodiment, a radiofrequency sum and a radiofrequency difference. A sum and a difference may be achieved, e.g., by multiplying the input and output signals. Since a multiplication between two frequencies is equivalent to a frequency sum and a frequency difference, the mix signal is composed of the desired radiofrequency sum and radiofrequency difference.

One ordinarily skilled in the art would appreciate that the advantage in the production of a radiofrequency sum and a radiofrequency difference is that whereas the radiofrequency sum includes both the signal and a considerable amount of electrical noise, the radiofrequency difference is approximately noise-free.

According to a preferred embodiment of the method continues to step **30** in which a portion of the mixed signal is filtered out such that a remaining portion of the mixed signal is characterized by a signal-to-noise ratio (SNR) which is substantially higher compared to the signal-to-noise ratio of the mixed signal or input radiofrequency signal.

The method continues to step **23** in which a phase shift Δ*ϕ* of the input radiofrequency signals relative to the output radiofrequency signals is determined. It was found by the inventors of the present invention that the phase shift of the input signals, as received from the subject, relative to the output signals as generated by the radiofrequency generator, is indicative of the cardiac output of the subject.

The advantage of using Δ*ϕ* for determining the cardiac output is that the relation between the blood flow and Δ*ϕ* depends on fewer measurement-dependent quantities as compared to conventional determination techniques in which the impedance is used. The phase shift can be determined for any frequency component of the spectrum of radiofrequency signals received from the organ. For example, in one embodiment, the phase shift is preferably determined from the base frequency component, in another embodiment the phase shift is preferably determined from the second frequency component, and so on. Alternatively the phase shift can be determined using several frequency components, e.g., using an appropriate averaging algorithm.

In some embodiments of the present invention the method continues to step **34** in which a dynamically variable filter is applied. The dynamically variable filter filters the data according to a frequency band which is dynamically adapted in response to a change in the physiological condition of the subject. It was found by the Inventor of the present invention that the dynamical adaptation of the frequency band to the physiological condition of the subject can significantly reduce the influence of unrelated signals on the measured property.

Thus, in the present embodiment, step **34** includes a process in which first the physiological condition of the subject is determined, then a frequency band is selected based on the physiological condition of the subject, and thereafter the input signals are filtered according to frequency band. The frequency band is dynamically adapted in response to a change in the physiological condition.

The physiological condition is preferably, but not obligatorily, the heart rate of the subject. The data pertaining to the physiological condition can be collected via a suitable data collection unit either in analog or digital form, as desired. For example, the physiological condition can be a heart rate which can be determined, *e.g*., by analysis of ECG signals or the like.

While the embodiments below are described with a particular emphasis to physiological condition which is a heart rate, it is to be understood that more detailed reference to the heart rate is not to be interpreted as limiting the scope of the invention in any way. For example, in exemplary embodiments of the present invention the physiological condition is a ventilation rate of the subject, a repetition rate of a particular muscle unit and/or one or more characteristics of an action potential sensed electromyography.

The adaptation of the frequency band to the physiological condition can be according to any adaptation scheme known in the art. For example, one or more parameters of the frequency band (*e.g*., lower bound, upper bound, bandwidth, central frequency) can be a linear function of a parameter characterizing the physiological condition. Such parameter can be, for example, the number of heart beats per minute.

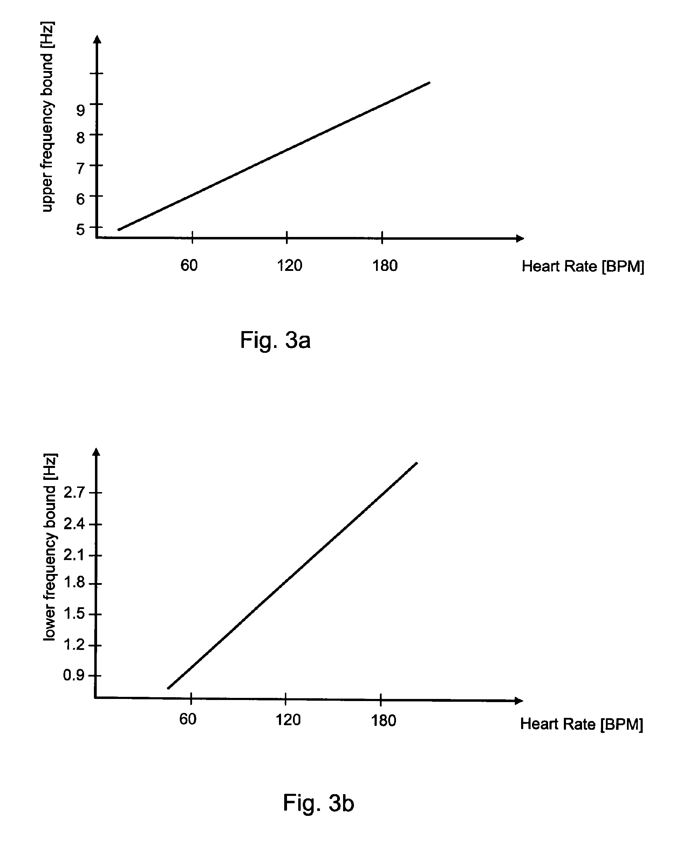

A representative example of a dynamically varying frequency bounds is illustrated in Figures 3a-b. Shown in Figures 3a-b is the functional dependence of the frequency bounds (upper bound in Figure 3a and lower bound in Figure 3b) on the heart rate of the subject. As shown in Figure 3a, the upper bound of the frequency band varies linearly such that at a heart rate of about 60 beats per minute (bpm) the upper bound is about 6 Hz, and at a heart rate of about 180 bpm the upper bound is about 9 Hz. Preferably, the upper bound is about 6 + 1.5 × [(HR/60) - 1]Hz at all times, where HR is the heart rate of the subject in units of bpm. As shown in Figure 3b, the lower bound of the frequency band varies linearly such that at a heart rate of about 60 the lower bound is about 0.9 Hz bpm and at a heart rate of about 180 bpm the lower bound is about 2.7 Hz. The lower bound is about 0.9 × (HR/60) Hz at all times.

As used herein the term "about" refers to ± 10 %.

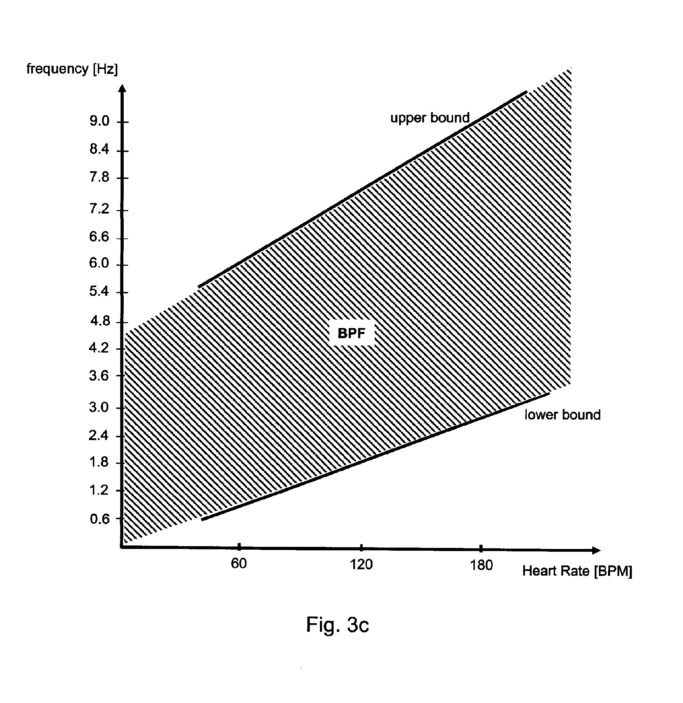

A dynamically varying band pass filter (BPF) characterized by the frequency bounds described above is illustrated in Figure 3c. As shown, each heart rate is associated with a frequency band defined by a lower bound and an upper bound. For example, for a heart rate of 60 bpm, Figure 3c depicts a BPF in which the lower bound is about 0.9 Hz and the upper bound is about 6 Hz.

It is to be understood that the values presented above and the functional relations illustrated in Figures 3a-b are exemplary embodiments and should not be considered as limiting the scope of the present invention in any way. In other exemplary embodiments, the functional relations between the frequency band and the physiological condition can have different slopes and/or offsets, or they can be nonlinear.

The method continues to step **36** in which the cardiac output is calculated, based on Δ*ϕ.* It was found by the inventor of the present invention that there is a linear relationship between Δ*ϕ* and the cardiac output, with a proportion coefficient comprising the systolic ejection time, *T*. For example, the cardiac output CO can be calculated using the relation CO = const.×*T*×Δ*ϕ*×HR, where HR is the heart rate of the subject (*e.g*., in units of beats per minutes), and "const." is a constant which can be found, for example, using a calibration curve.

The method continues to step **38** in which the calculated cardiac output is used for estimating the exercise capacity of the subject. Generally, the exercise capacity correlates with the cardiac output. For example, when the cardiac output is below a predetermined threshold, the method can estimate that the subject's exercise capacity is low, and when the cardiac output is above a predetermined threshold, the method can estimate that the subject's exercise capacity is high. It was demonstrated by the present inventors that during exercise the cardiac output among normal subjects is about 34 % higher than that of CHF patients. The method of the present embodiments can therefore be used to assess or determine worsening of the condition of the subject, particularly subjects with congestive heart failure.

In some embodiments of the present invention, the method continues to step **40** in which a cardiopulmonary exercise testing is performed to provide one or more CPX. The cardiac output can be combined with the CPX measure(s) and the combination can be used to estimate the exercise capacity, and/or to assess the quality of the estimation. For example, as demonstrated in the Examples section that follows, the maximal cardiac output is inversely correlated to the VE/VCO₂ slope, where VE is the ventilation efficiency and VCO₂ is the carbon dioxide production rate. The correlation coefficient between the maximal cardiac output during exercise and the VE/VCO₂ slope can be calculated and the quality of the exercise capacity estimation can be assessed based on this correlation coefficient, where negative and large in absolute value correlation coefficient corresponds to high quality of exercise capacity estimation and vice versa.

It is further demonstrated in the Examples section that follows that the maximal cardiac output is directly correlated to the oxygen uptake efficiency slope OUES. The correlation coefficient between the maximal cardiac output during exercise and the OUES can be calculated and the quality of the exercise capacity estimation can be assessed based on this correlation coefficient, where high positive correlation coefficient corresponds to high quality of exercise capacity estimation and vice versa.

The method ends at step **42.**

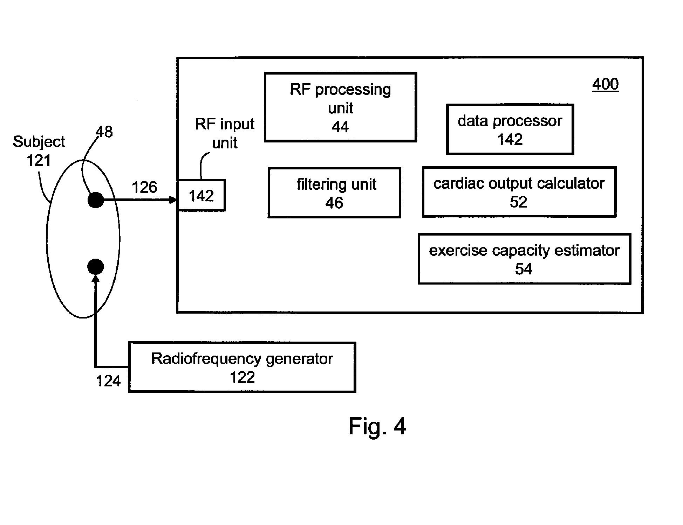

Reference is now made to Figure 4 which is a schematic illustration of apparatus **400** for estimating exercise capacity of a subject **121,** according to various exemplary embodiments of the present invention.

Apparatus **400** comprises an input unit **142** for receiving input radiofrequency signals sensed from the organ. The input radiofrequency signals typically comprise radiofrequency signals related to the electrical properties of the organ (*e.g*., bioimpedance which may generally relate to the impedance and/or hemodynamic reactance of the organ). The signals are sensed from one or more sensing locations **48** on the organ of subject **121** and are originated by output radiofrequency signals **124** generated by a radiofrequency generator **122.**

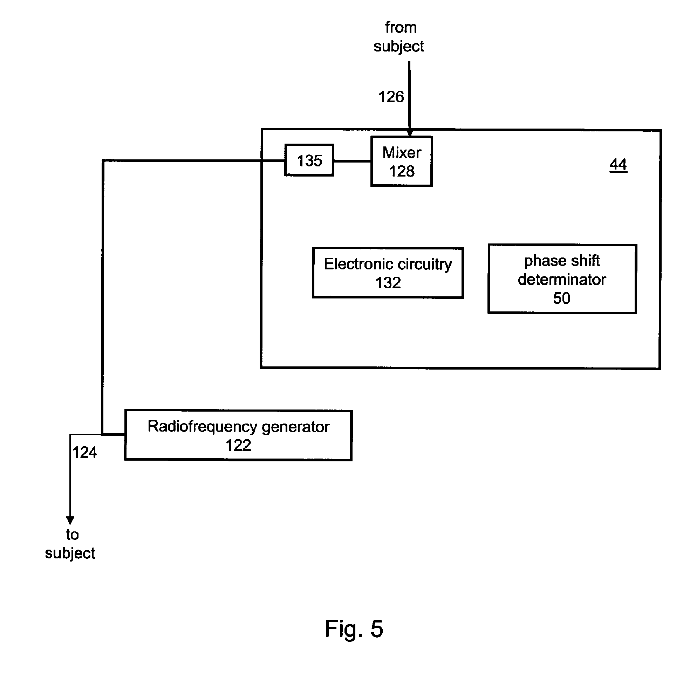

Apparatus **400** further comprises a signal processing unit **44** which processes the input radiofrequency signals. The processing may include, for example, mixing, demodulation, determination of phase shift, analog filtering, sampling and any combination thereof. Signal processing unit **44** may or may not be in communication with radiofrequency generator **122,** as desired. A representative example of signal processing unit **44** is provided hereinunder with reference to Figure 5.

Apparatus **400** is designed for determining a phase shift Δ*ϕ* of signals **126** relative to signals 124. This can be done using a phase shift determinator **50** (not shown, see Figure 5) which can operate according to any known technique for determining a phase shift. The phase shift can be determined for any frequency component of the spectrum of radiofrequency signals received from the organ. For example, in one embodiment, the phase shift is determined from the base frequency component, in another embodiment the phase shift is determined from the second frequency component, and so on. Alternatively the phase shift can be determined using several frequency components, *e.g*., using an appropriate averaging algorithm.

The input radiofrequency signals may include one or more noise components, which may be introduced into the signal due to various reasons, *e.g*., subject agitation or breathing. In various exemplary embodiments of the invention apparatus **400** is capable of reducing or eliminating these noise components. In some embodiments of the present invention apparatus **400** further comprises a filtering unit **46** which filters the processed input signals. Unit **46** preferably performs the filtration operation in the frequency domain. Thus, in various exemplary embodiments of the invention, a series of samples of the processed radiofrequency signals are transformed, e.g., by a Fast Fourier Transform (FFT), to provide a spectral decomposition of the signals in the frequency domain. The transformation to the frequency domain can be done by a data processor. Algorithms for performing such transformations are known to those skilled in the art of signal processing.

The obtained spectral decomposition of the signal is filtered by unit **46** which typically eliminates one or more of the frequencies in the spectrum, depending on the upper and lower frequency bounds of the filter employed by unit **46.** Unit **46** preferably employs a dynamically variable filter, such as, but not limited to, the dynamically variable filer described hereinabove.

According to the present invention apparatus **400** comprises a cardiac output calculator **52** which calculates the cardiac output and an exercise capacity estimator **54** which uses the calculated cardiac output for estimating the exercise capacity of the subject as further detailed hereinabove. In some embodiments, exercise capacity estimator **54** combines the calculated cardiac output with one or more CPX measures, *e.g*., by means of performing statistical analysis. Cardiac output calculator **52** and exercise capacity estimator **54** can be associated with a data processor **142.** Data processor **142** can also be employed by unit **46** for performing the transformation to the frequency domain and/or eliminating the frequency components according to the dynamically variable frequency bounds.

Data processor **142** can also be configured for calculating other quantities, *e.g*., stroke volume and/or other blood-volume related quantities.

Reference is now made to Figure 5 which schematically illustrates signal processing unit **44,** according to various exemplary embodiments of the present invention. Unit **44** preferably comprises a mixer **128,** electrically communicating with generator **122,** for mixing output signals **124** and input signals **126,** so as to provide a mixed radiofrequency signal. Signals **124** and **126** may be inputted into mixer **128** through more than one channel, depending on optional analog processing procedures (*e.g*., amplification) which may be performed prior to the mixing.

Mixer **128** may be any known radiofrequency mixer, such as, but not limited to, double-balanced radiofrequency mixer and unbalanced radiofrequency mixer. According to a preferred embodiment of the present invention, the mixed radiofrequency signal is composed of a plurality of radiofrequency signals, which may be, in one embodiment, a radiofrequency sum and a radiofrequency difference. A sum and a difference may be achieved, *e.g.*, by selecting mixer **128** such that signals **124** and signals **126** are multiplied thereby. Since a multiplication between two frequencies is equivalent to a frequency sum and a frequency difference, mixer **128** outputs a signal which is composed of the desired radiofrequency sum and radiofrequency difference.

According to various exemplary embodiments of the present invention unit **44** further comprises a phase shift determinator **50** for determining the phase shift of the input signals relative to the output signal. Phase shift determinator **50** can determine the phase shift according to any technique known in the art. For example, the phase shift can be determined from the radiofrequency difference outputted from mixer **128.**

According to a preferred embodiment of the present invention processing unit **44** further comprises electronic circuitry **132,** which filters out a portion of the signal such that a remaining portion of the signal is characterized by a substantially increased signal-to-noise ratio.

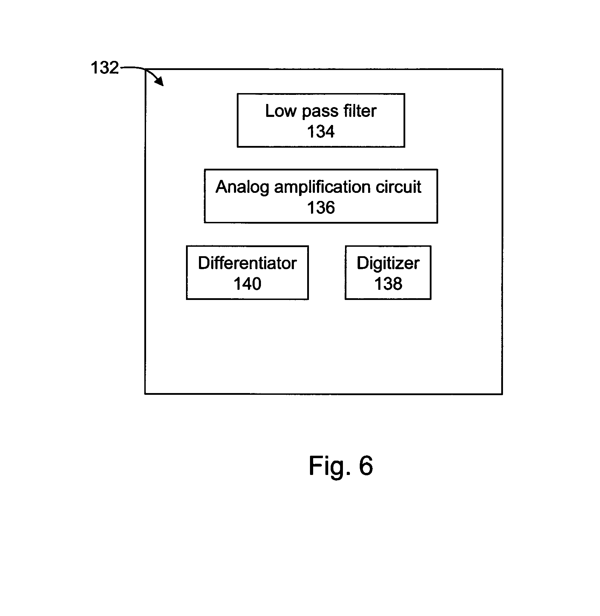

Circuitry **132** is better illustrated in Figure 6. According to an embodiment of the present invention circuitry **132** comprises a low pass filter **134** to filter out the high frequency content of the signal. Low pass filter **134** is particularly useful in the embodiment in which mixer **128** outputs a sum and a difference, in which case low pass filter **134** filters out the radiofrequency sum and leaves the approximately noise-free radiofrequency difference. Low pass filter **134** may be designed and constructed in accordance with the radiofrequency difference of a particular system which employs apparatus **400.** A judicious design of filter **134** substantially reduces the noise content of the remaining portion.

Circuitry **132** preferably comprises an analog amplification circuit **136** for amplifying the remaining portion of the signal. The construction and design of analog amplification circuit **136** is not limited, provided circuit **136** is capable of amplifying the signal. Amplification circuits suitable for the present embodiments are found in International Patent Application, Publication Nos. WO 2004/098376 and WO 2006/087696.

According to a preferred embodiment of the present invention circuitry **132** further comprises a digitizer **138** for digitizing the signal. The digitization of the signal is useful for further digital processing of the digitized signal, *e.g.,* by a microprocessor.

Optionally, circuitry comprises a differentiator **140** (either a digital differentiator or an analog differentiator) for performing at least one time-differentiation of the measured impedance to obtain a respective derivative (*e.g*., a first derivative, a second derivative, *etc.*) of the bioimpedance or hemodynamic reactance. Differentiator **140** may comprise any known electronic functionality (*e.g*., a chip) that is capable of performing analog or digital differentiation.

According to a preferred embodiment of the present invention signal processing unit **44** comprises an envelope elimination unit **135** which reduces or, more preferably, eliminates amplitude modulation of signals **126.** Optionally and preferably, unit **135** maintains the phase modulation of signals **126.** The output of unit **135** represents the phase (or frequency) modulation of signal **126,** as further detailed hereinabove. Unit **135** can employ, for example, a limiter amplifier which amplifies signals **126** and limits their amplitude such that the amplitude modulation is removed. The advantage of the removal of the amplitude modulation is that it allows a better determination of the phase shift Δ*ϕ* between the input and output signals, as further detailed hereinabove.

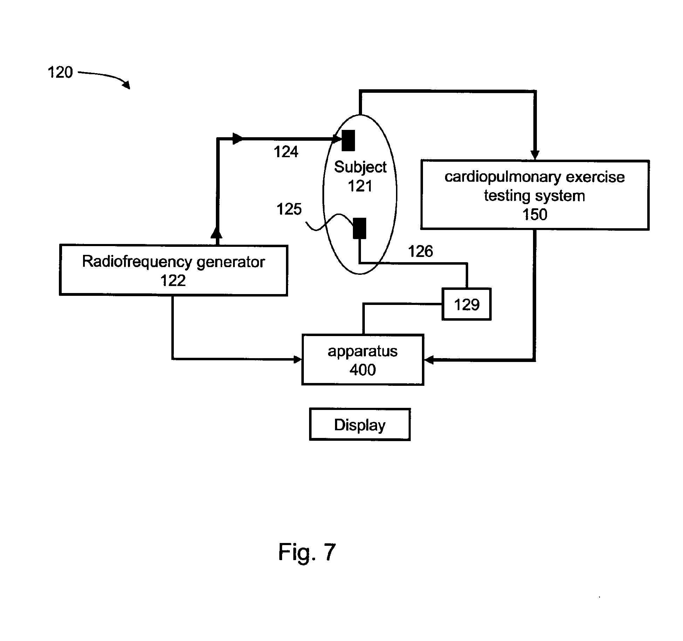

Reference is now made to Figure 7, which is a schematic illustration of system **120** for estimating exercise capacity of a subject, according to a preferred embodiment of the present invention. System **120** preferably comprises a radiofrequency generator **122,** for generating output radiofrequency signals. Generator **122** may be embodied as any radiofrequency generator. System **120** further comprises a plurality of electrodes **125,** which are connected to the skin of subject **121.** Electrodes **125** transmit output radiofrequency signals **124,** generated by generator **122** and sense input radiofrequency signals **126** originated from the organ of subject **121.**

System **120** comprises any of the components of apparatus **400** described above. According to a preferred embodiment of the present invention system **120** further comprises a detector **129** for detecting a voltage drop on a portion of the body of subject **121** defined by the positions of electrodes **125.** In response to the detected voltage, detector **129** preferably generates signals which are indicative of impedance of the respective portion of the body. In this embodiment, the stroke volume can be calculated using (*dX*/*dt*)ₘₐₓ*,* as further detailed hereinabove. Knowing the stroke volume, the cardiac output is calculated by multiplying the stroke volume by the heart rate of the subject. More preferably, detector **129** generates signals which are indicative of a hemodynamic reactance, *X*.

In some embodiments, system **120** comprises a cardiopulmonary exercise testing system **150** which provides apparatus **400** with one or more CPX measure via a communication line **152.** Apparatus **400** combines the calculated cardiac output with the CPX measure(s) for estimating the exercise capacity as further detailed hereinabove.

Following are technical preferred values which may be used for selective steps and parts of the embodiments described above.

The output radiofrequency signals are preferably from about 10 KHz to about 200 KHz in frequency and from about 10mV to about 200mV in magnitude; the input radiofrequency signals are preferably about 75 KHz in frequency and about 20mV in magnitude; a typical impedance which can be measured by the present embodiments is from about 5 Ohms to about 75 Ohms; the resulting signal-to-noise ratio of the present embodiments is at least 40dB; low pass filter **134** is preferably characterized by a cutoff frequency of about 35Hz and digitizer **138** preferably samples the signals at a rate of about 500-1000 samples per second.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following example.

### EXAMPLES

Reference is now made to the following example, which together with the above descriptions illustrates some embodiments of the invention in a non limiting fashion.

Some embodiments of the present invention were utilized for the purpose of estimating the exercise capacity of subjects.

### Methods

### Subjects

Data obtained from 36 consecutive subjects referred to a private cardiology clinic for CPX testing for evaluation of dyspnea. All patients provided consent for the use of their data in the analysis. Twenty three of the subjects had heart failure (18 with low EF, 5 with normal EF) and 13 were ultimately diagnosed as normal (normal EF and peak VO₂, dyspnea based on noncardiac factors). Demographic and clinical characteristics of the subjects are summarized in Table 1.

**Table 1**

| **Patient Characteristics** | **CHF** | **Normal** | **P** |
|---|---|---|---|
| N | 23 | 13 | |
| Age (years) | 66.6 ± 10 | 51.0 ± 11 | <0.001 |
| Height (cm) | 176 ± 10 | 172 ± 10 | 0.20 |
| Weight (kg) | 87.0 ± 15 | 76.1 ± 15 | 0.04 |
| Ejection Fraction (%) | 35.9 ± 13 | 56.5 ± 9.2 | 0.003 |
| Peak VO₂ (ml/kg/min) | 14.7 ± 5.4 | 24.8 ± 6.2 | <0.001 |
| CHF Etiology, # subjects (%) | | | |
| Ischemic Cardiomyopathy | 17 (74) | - | - |
| Idiopathic Dilated Cardiomyopathy | 1 (4) | - | - |
| CHF with Normal EF* | 4 (17) | - | - |
| NYHA Classification, # (%) | | - | |
| Class I | 1 (5) | - | - |
| Class II | 3 (15) | - | - |
| Class III | 13 (65) | - | - |
| Class IV | 3 (15) | - | - |
| Medications, # subjects (%) | | | |
| Digoxin | 3 (13) | 0 (0) | - |
| Beta Blocker | 22 (96) | 6 (46) | 0.05 |
| ACE/ARB | 17 (74) | 5 (38) | 0.05 |

| | | | |
|---|---|---|---|
| CHF=chronic heart failure; EF=ejection fraction; NYHA= New York Heart Association; ACE/ARB=ACE inhibitor/angiotensin receptor blocker; * > 45 % | | | |

In the overall population there was a broad range of EFs, peak VO₂ values and peak cardiac outputs. All subjects were limited during exercise by fatigue or dyspnea, and none had clinical evidence of pulmonary disease or ischemic changes on the ECG.

### Exercise Testing

Symptom limited maximal exercise tests were performed on a treadmill using a ramp protocol [Myers J, Buchanan N, Walsh D, Kraemer M, McAuley P, Hamilton-Wessler M, Froelicher VF. Comparison of the ramp versus standard exercise protocols, J Am Coll Cardio 1991;17:1334-1342].

All subjects were requested to abstain from eating or smoking at least 3 hours prior to the test. Ventilatory oxygen uptake was measured using a Medical Graphics Corporation (CPX-D, St. Paul, MN). Gas exchange data were acquired breath-by-breath and expressed in ten second intervals of rolling 30 second averages. Oxygen uptake, carbon dioxide production, minute ventilation and respiratory exchange ratio were calculated on-line. The percentage of age-predicted normal peak VO₂ was determined for each patient using the equation of Wasserman et al. [Wasserman K, Hansen JE, Sue DY, Casaburi R, Whipp BJ. Principles of exercise testing and interpretation, 4th ed. Baltimore: Lippincott, Williams & Wilkins, 2004]. Estimated peak VO₂ was determined from the American College of Sports Medicine equations [American College of Sports Medicine. Guidelines on Exercise Testing and Prescription, 7th ed. Baltimore: Lippincott, Williams & Wilkins, 2006]. A 12-lead electrocardiogram was monitored continuously and recorded every minute. Blood pressure was recorded manually every two minutes throughout the test.

All subjects were encouraged to provide a maximal effort; among patients with CHF, the Borg 0 to 10 perceived exertion scale was used to quantify effort [Borg GAV. Borg's perceived exertion scales, Champaign: Human Kinetics, 1998]. The ventilatory threshold was determined by two experienced, independent reviewers using the V-slope method [Beaver WL, Wasserman K, Whipp BJ. A new method for detecting the anaerobic threshold by gas exchange. J Appl Physiol 1986;60:2020-2027] and confirmed by ventilatory criteria. Ventilatory efficiency (VE) and VCO₂ responses, acquired from the initiation of exercise to peak, were used to calculate a VE/VCO₂ slope via least squares linear regression. Oxygen uptake efficiency slope (OUES) was derived by the slope of a semi-log plot of minute ventilation versus VO₂. As such, the OUES is an estimation of the efficiency of ventilation with respect to VO₂, with greater slopes indicating greater ventilatory efficiency.

### Cardiac Output

Cardiac output was determined using a NICOM system (Cheetah Medical, Wilmington, Delaware) disclosed in WO 2006/087696 and supplemented by a dynamically variable filter with an upper bound of 6 + 1.5 × [(HR/60) - 1] Hz and a lower bound of 0.9 × (HR/60) Hz, as described above. The system included 4 dual surface electrodes, where each dual electrode included one electrode for transmitting the radiofrequency output signals and one electrode for receiving the input signals. The signals were applied to and recorded from the left and right sides of the thorax; the signals were processed separately and averaged after digital processing.

The signal processing unit of the system determined the phase shift Δ*ϕ* of the input radiofrequency signals relative to the output radiofrequency signals, applied the dynamically variable filter and calculated the cardiac output as further detailed hereinabove.

Nine subjects with Coronary Artery Disease (CAD) who underwent maximal exercise testing with simultaneous measurement of cardiac output using the direct Fick method are included for comparison purposes.

### Statistical Analysis

Descriptive statistics are presented below as mean ± standard deviation (SD). The associations between non-invasive cardiac output data, clinical variables and other exercise test responses were assessed using linear regression.

### Results

### Exercise Test Responses

Exercise test responses are summarized in Table 2.

**Table 2**

| | **CHF** | **Normal** | **p value** |
|---|---|---|---|
| **Rest** | | | |
| Standing heart rate (beats/min) | 73 ± 13 | 75 ± 13 | 0.72 |
| Systolic blood pressure (mmHg) | 121 ± 9 | 125 ± 9 | 0.23 |

| **Ventilatory Threshold** | | | |
|---|---|---|---|
| Heart rate (beats/min) | 95 ± 28 | 136 ± 27 | <0.001 |
| Systolic blood pressure (mmHg) | 131 ± 12 | 139 ± 9 | 0.06 |
| Diastolic blood pressure (mmHg) | 80 ± 9 | 83 ± 8 | 0.48 |
| Oxygen uptake (ml/min) | 1046 ± 501 | 1517 ± 493 | 0.01 |
| Oxygen uptake (ml/kg/min) | 11.9 ± 4.4 | 20.9 ± 7.4 | <0.001 |
| Minute ventilation (l/min) | 32.0 ± 13.0 | 39.3 ± 13.8 | 0.14 |
| CO₂ production (ml/min) | 1005 ± 512 | 24.8 ± 590 | 0.02 |
| Exercise time (min) | 24.8 ± 4.2 | 5.6 ± 3.1 | 0.23 |
| Perceived exertion | 24.8 ± 2.8 | - | - |

| **Maximal Exercise** | | | |
|---|---|---|---|
| Heart rate (beats/min) | 24.8 ± 24 | 139 ± 24 | <0.001 |
| Systolic blood pressure (mmHg) | 134 ± 14 | 143 ± 12 | 0.04 |
| Oxygen uptake (ml/min) | 1310 ± 587 | 1857 ± 464 | 0.007 |
| Oxygen uptake (ml/kg/min) | 14.7 ± 5.4 | 24.8 ± 6.1 | <0.001 |
| Minute ventilation (l/min) | 49.4 ± 19.8 | 63.7 ± 16.5 | 0.03 |
| CO₂ production | 1442 ± 660 | 2173 ± 577 | 0.01 |
| Respiratory exchange ratio | 1.10 ± 0.08 | 1.15 ± 0.09 | 0.13 |
| Exercise time (min) | 10.7 ± 4.4 | 9.1 ± 2.1 | 0.21 |
| Perceived exertion | 7.8 ± 1.8 | - | - |

For the overall group, the mean maximal perceived exertion was 24.8 ± 1.7 (range 5-10), and the mean peak respiratory exchange ratio was 1.12 ± 0.09 (range 0.87-1.28), suggesting that maximal effort was achieved by most patients. Among CHF patients, the mean maximal heart rate of 103 ± 24 beats/min (range 76-140) was lower than that expected for age (61 % of predicted), reflecting the fact that many patients were limited by symptoms associated with CHF, that heart rate was limited by the effects of beta-blockade, or both. Mean maximal oxygen uptake for the CHF sample was 14.7 ± 5.4 ml/kg/min (range 6.8-25.7) (representing 50.0 ± 21 % of age-predicted peak VO₂), and the ventilatory threshold occurred at 81 % of peak VO₂. The wide ranges of each of these parameters indicate that the sample was diverse, which was desired for this initial evaluation.

### Cardiac Output

Cardiac output measurements at rest and during exercise are presented in Table 3.

**Table 3**

| **Rest** | **CHF** | **Normals** | **p value** |
|---|---|---|---|
| Cardiac output (L/min) | 4.9 ± 1.5 | 5.7 ± 1.8 | 0.28 |
| Cardiac index (L/min/M²) | 2.5 ± 0.70 | 3.1 ± 0.7 | 0.06 |
| Dx/dt (ohme/sec) | 122.0 ± 84.9 | 196.1 ± 108.5 | 0.13 |
| VET (msec) | 159.7 ± 21.2 | 172.5 ± 35.3 | 0.12 |

| **Peak Exercise** | | | |
|---|---|---|---|
| Cardiac output (L/min) | 15.0 ± 6.6 | 20.1 ± 9.3 | 0.11 |
| Cardiac index (L/min) | 7.4 ± 3.0 | 11.0 ± 4.6 | 0.02 |
| Dx/dt (ohme/sec) | 377.2 ± 195.0 | 639.6 ± 188.2 | 0.01 |
| VET (msec) | 147.3 ± 21.2 | 141.3 ± 17.9 | 0.67 |

Among patients with CHF, cardiac output increased from 4.9 ± 1.5 to 15.0 ± 6.6 L/min at peak exercise, along with a concomitant increase in dΦ/dtₘₐₓ (122.0±84.9 to 377.2±195.0 ohme/sec) and an 8% reduction in VET. Cardiac output among normal subjects was 16% and 34% higher than that of CHF patients at rest and during exercise, respectively.

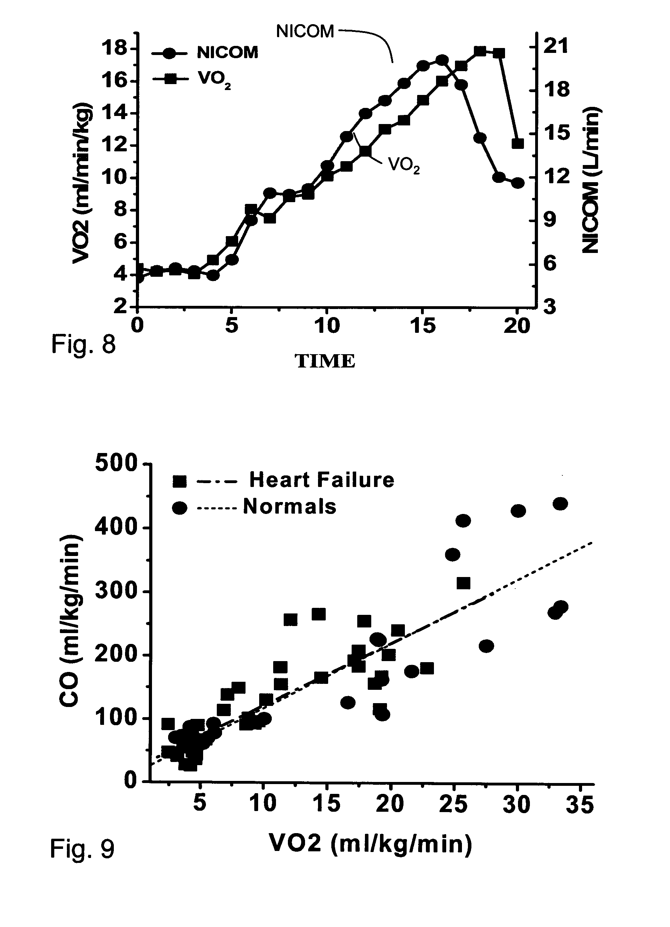

Figure 8 illustrates the changes in oxygen uptake superimposed with estimated cardiac output over time in a typical example, demonstrating that they closely paralleled one another during the test. The relationship between cardiac output and oxygen uptake at rest and peak exercise is presented in Figure 9. There is a strong association between cardiac output and oxygen uptake (r=0.89, p<0.001).

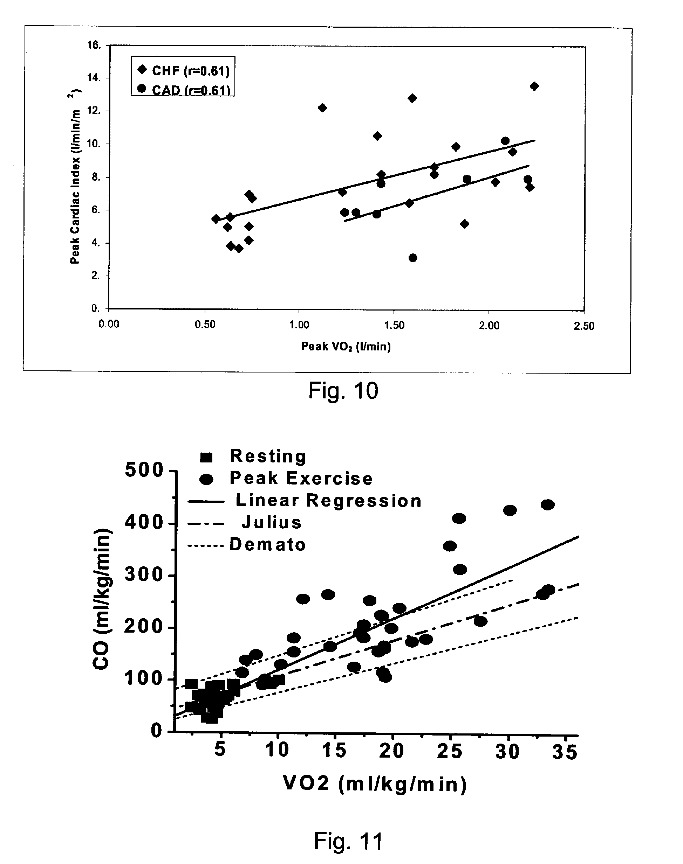

Figure 10 shows relationships between peak cardiac index and peak VO₂ in CHF patients tested according to the present embodiments and patients with CAD tested using the direct Fick method. The relationships between peak cardiac index and peak VO₂ were similar for the directly measured (r=0.61) and non-invasive (r=0.61) methods.

Figure 11 presents the association between resting and peak exercise cardiac output and oxygen uptake obtained according to the present embodiments. The association is superimposed with other studies [Julius S, Amery A, Whitlock LS, Conway J. Influence of age on the hemodynamic response to exercise. Circulation. 1967;36:222-230; and Damato AN, Galante JG, Smith WM. Hemodynamic response to treadmill exercise in normal subjects. J. Appl Physiol 1966;21:959-966]. The relation between cardiac output and oxygen uptake was similar between the current study and the two earlier studies, as evidenced by the similar line of best fit (current and Julius study) and similar 95 % confidence limits (current and Damato studies).

Table 3 below presents correlation coefficients between estimated cardiac output and key clinical and exercise test responses. In Table 3, values followed by an asterisk (*) refer to p<0.05, values followed by the pound sign (#) refer to p<0.01, CO refers to cardiac output in ml/kg/min, CI refers to cardiac index in L/min/m², dX/dt refers to the peak aortic flow, BP refers to blood pressure in mmHg, HR refers to heart rate, VET refers to ventricular ejection time in msec, refers to VO₂VT refers to oxygen uptake at the ventilatory threshold in ml/kg/min, and OUES refers to oxygen uptake efficiency slope.

**Table 3**

| | Rest CO | Rest CI | Rest dX/dt | Rest HR | Rest Syst. BP | Rest VET | Age | Eject. Frac. | Peak HR | Peak VO2 | VO₂ VT | VE/ VCO₂ | OUES | Max CO | Max CI | Max dX/dt |
|---|---|---|---|---|---|---|---|---|---|---|---|---|---|---|---|---|
| Rest CO | | | | | | | | | | | | | | | | |
| Rest CI | 0.96# | - | | | | | | | | | | | | | | |
| Rest dX/dt | 0.77# | 0.79# | - | | | | | | | | | | | | | |
| Rest HR | 0.54# | 0.57# | 0.37 | - | | | | | | | | | | | | |
| Rest Syst. BP | -0.27 | -0.22 | -0.23 | 0.00 | - | | | | | | | | | | | |
| Rest VET | 0.07 | -0.02 | -0.32 | -0.13 | 0.07 | - | | | | | | | | | | |
| Age | -0.27 | -0.28 | -0.16 | -0.07 | 0.01 | -0.15 | - | | | | | | | | | |
| Eject. Frac. | 0.04 | -0.04 | -0.10 | 0.17 | 0.28 | 0.38* | -0.24 | - | | | | | | | | |
| Peak HR | 0.48# | 0.48# | 0.20 | 0.36* | 0.09 | 0.37* | -0.49# | 0.35* | - | | | | | | | |
| Peak VO₂ | 0.36* | 0.28 | 0.18 | 0.21 | 0.05 | 0.34* | -0.52# | 0.41* | 0.67# | - | | | | | | |
| VO₂ VT | 0.24 | 0.15 | 0.07 | 0.04 | 0.09 | 0.36* | -0.58# | 0.42* | 0.56# | 0.91# | - | | | | | |
| VE/ VCO₂ | -0.04 | -0.08 | -0.09 | -0.09 | -0.37* | -0.03 | 0.56# | -0.09 | -0.41# | -0.59# | -0.61# | - | | | | |
| OUES | 0.19 | 0.26 | -0.16 | 0.13 | 0.11 | 0.20 | -0.48# | 0.15 | 0.55# | 0.81# | 0.76# | -0.66# | - | | | |
| Max CO | 0.18 | 0.17 | -0.05 | 0.10 | 0.13 | 0.34* | -0.58# | 0.19 | 0.60# | 0.73# | 0.74# | -0.47# | 0.67# | - | | |
| Max CI | 0.12 | 0.18 | -0.06 | 0.09 | 0.17 | 0.28 | -0.55# | 0.09 | 0.55# | 0.64# | 0.66# | -0.48# | 0.72# | 0.97# | - | |
| Max dX/dt | 0.48# | 0.49# | 0.30 | 0.19 | -0.02 | 0.08 | -0.61# | 0.00 | 0.46# | 0.55# | 0.63# | -0.39* | 0.50# | 0.79# | 0.78# | - |
| Max Syst. BP | -0.01 | 0.06 | -0.16 | 0.04 | 0.60# | 0.36* | -0.06 | 0.36* | 0.35* | 0.33* | 0.21 | -0.29 | 0.40* | 0.24 | 0.29 | 0.06 |

Both maximal cardiac output and cardiac index were strongly related to peak VO₂ (r=0.73 and 0.64, respectively, p<0.001). Peak VO₂ was modestly associated with resting cardiac output (r=0.36 and 0.28 for resting cardiac output and cardiac index, respectively), and resting estimates of parameters related to ventricular contractility (0.41 and 0.34 for EF and VET, respectively). Maximal dΦ/dt was strongly associated with both maximal cardiac output and cardiac index (r=0.79 and 0.78, respectively, p<0.001).

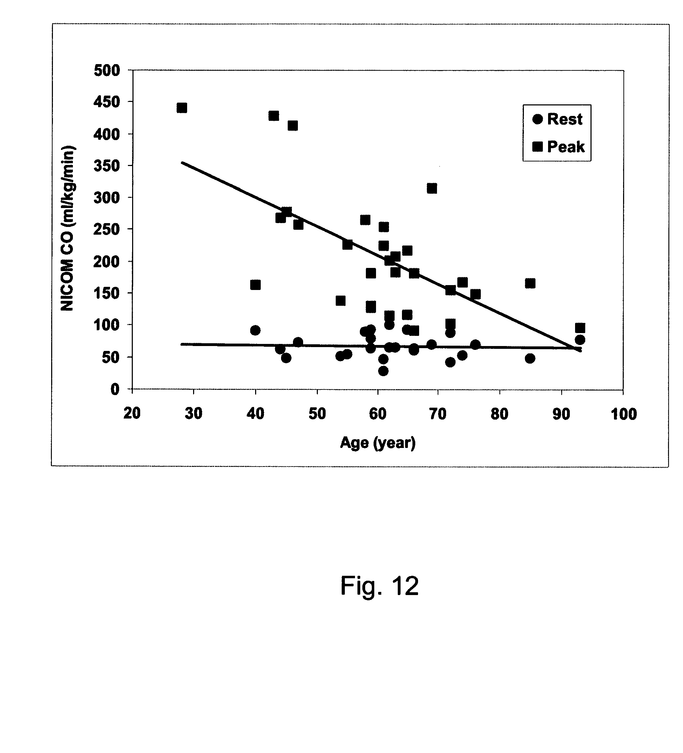

Figure 12 shows the relationship between cardiac output as calculated according to some embodiments of the present invention and age. Shown in Figure 12 are cardiac output values measured at rest (circles) and peak exercise (squares). Resting cardiac output was weakly associated with age (r=-0.27, ns), but a significant inverse association was observed at peak exercise (r=-0.58, p<0.001).

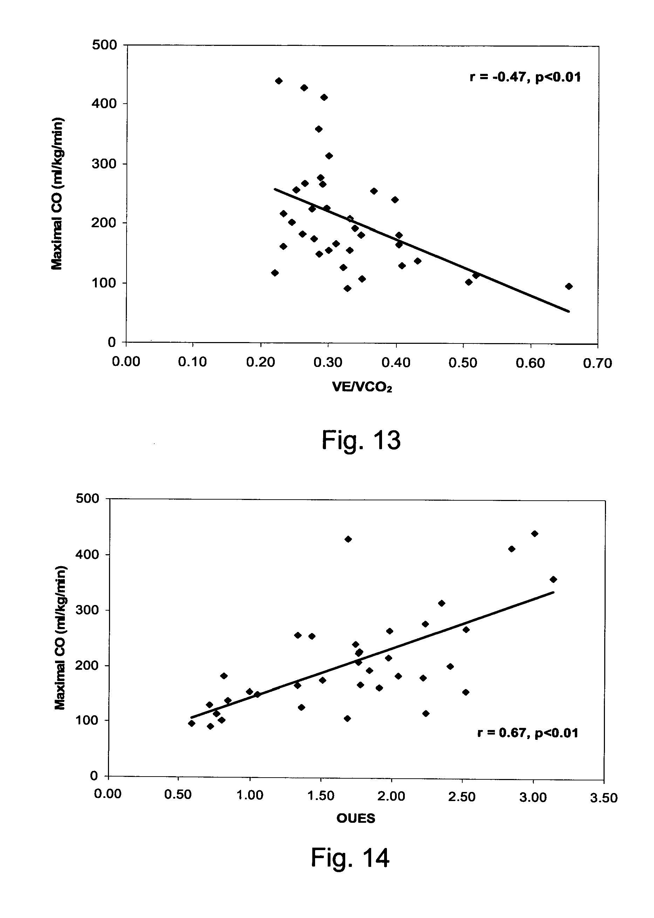

Figure 13 shows the relationship between maximal cardiac output as calculated according to some embodiments of the present invention and the VE/VCO₂ slope. Maximal cardiac output was inversely related to the VE/VCO₂ slope (r=-0.47, p<0.01). The VE/VCO₂ slope was also found to be have strong relation to VO₂ at peak exercise (r=-0.59, p<0.01) and VO₂ at the ventilatory threshold (r=-0.61, p<0.01).

Figure 14 shows the relationship between maximal cardiac output as calculated according to some embodiments of the present invention and the OUES. Maximal cardiac output was directly related to the OUES (r=0.67, p<0.01). The OUES was also found to be have strong relation to VO₂ at peak exercise (r=0.81, p<0.001) and VO₂ at the ventilatory threshold (r=0.76, p<0.001).

### Discussion

The present example demonstrated the ability of some embodiments of the present invention to calculate cardiac output during exercise so as to estimate exercise capacity. A linear association between cardiac output as calculated according to some embodiments of the present invention and VO₂ at rest and during exercise has been demonstrated (r=0.89, p<0.001, Figure 9). The calculated cardiac output and VO₂ closely paralleled one another throughout exercise (Figure 18). Associations between peak VO₂ and peak calculated cardiac output (r=0.73) and peak cardiac index (r=0.64) were also observed. The relationships between peak cardiac index and peak VO₂ were similar for the directly measured (r=0.61) and non-invasive (r=0.61) methods (Figure 10). Relatively modest associations were observed between resting cardiac output and exercise performance and direct association between the various estimates of cardiac performance and VO₂ at the ventilatory threshold were observed (Table 3).

The results presented in this example demonstrate that the present embodiments provide face validity for estimating exercise capacity, particularly, but not exclusively, in patients with CHF.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. A method (10, 20) of estimating exercise capacity of a subject using output radiofrequency signals (124) transmitted to the subject during exercise and input radiofrequency signals (125) received from the subject during exercise, the method (10, 20) comprising:
processing said input radiofrequency signal, wherein said processing comprises determining (12) a phase shift of the input radiofrequency signals (125) relative to the output radiofrequency signals (124), to provide a processed input signal, which comprises said phase shift;
wherein the method (10, 20) further comprises:
filtering (34) said processed signal using a dynamically variable band pass filter **characterized by** a frequency band which is dynamically adapted in response to a change in a physiological condition of the subject, thereby providing a filtered signal;
calculating (14) cardiac output based on said phase shift; and
using (16) said cardiac output for estimating the exercise capacity of the subject.

2. The method (10, 20) of claim 1, further comprising obtaining (40) at least one CPX measure pertaining to a cardiopulmonary exercise testing, and combining said cardiac output with said at least one CPX measure to estimate the exercise capacity of the subject.

3. The method (10, 20) of claim 1, further comprising reducing or eliminating (26) amplitude modulation of said input radiofrequency signals (125) so as to provide input radiofrequency signals (125) of substantially constant envelope.

4. The method (10, 20) of claim 1, further comprising mixing said output radiofrequency signals (124) and said input radiofrequency signals (125) so as to provide a mixed radiofrequency signal, and filtering out a portion of said mixed radiofrequency signal so as to substantially increase a signal-to-noise ratio of a remaining portion of said mixed radiofrequency signal.

5. Apparatus (400) for estimating exercise capacity of a subject using output radiofrequency signals (124) transmitted to the subject during exercise and input radiofrequency signals (125) received from the subject during exercise, the apparatus (400) comprising:
a phase shift determinator (50) configured for determining a phase shift of the input radiofrequency signals (125) relative to the output radiofrequency signals (124), to provide a processed signal, which comprises said phase shift;
wherein the apparatus (400) further comprises:
a filtering unit (46) configured for filtering said processed signal using a dynamically variable band pass filter **characterized by** a frequency band which is dynamically adapted in response to a change in a physiological condition of the subject, to thereby provide a filtered signal; and
a cardiac output calculator (52) configured for calculating cardiac output based on said phase shift; and
an exercise capacity estimator (54) configured for using said cardiac output for estimating the exercise capacity of the subject.

6. The apparatus (400) of claim 5, wherein said exercise capacity estimator (54) is configured for combining said cardiac output with at least one CPX measure to estimate the exercise capacity of the subject.

7. A system (120) for estimating exercise capacity of a subject, comprising:
a radiofrequency generator (122) for generating output radiofrequency signals (124);
a plurality of electrodes (125) designed for transmitting said output radiofrequency signals (124) to the subject and for sensing input radiofrequency signals (125) from the subject; and
the apparatus (400) of claim 5.

8. The system (120) of claim 7, further comprising a cardiopulmonary exercise testing system (150) configured to provide at least one CPX measure, wherein said exercise capacity estimator (54) is configured for combining said cardiac output with at least one CPX measure to estimate the exercise capacity of the subject.

9. The apparatus (400) or system (120) of claim 5 or 7, wherein the apparatus (400) further comprises an envelope elimination unit (135) designed and configured for reducing or eliminating amplitude modulation of said input radiofrequency signals so as to provide input radiofrequency signals (125) of substantially constant envelope.

10. The apparatus (400) or system (120) of claim 5 or 7, wherein the apparatus (400) further comprises:
a mixer (128) configured for mixing said output radiofrequency signals (124) and said input radiofrequency signals (125), to provide a mixed radiofrequency signal; and
a radiofrequency filter (132) for filtering out a portion of said mixed radiofrequency signal so as to substantially increase a signal-to-noise ratio of a remaining portion of said mixed radiofrequency signal.

11. The method (10, 20), apparatus (400) or system (120) of claim 1, 5 or 7, wherein said cardiac output is calculated using a linear relationship between said phase shift and said cardiac output.

12. The method (10, 20), apparatus (400) or system (120) of claim 1, 5 or 7, wherein said physiological condition is a heart rate of the subject.

13. The method (10, 20), apparatus (400) or system (120) of claim 12, wherein said filter is **characterized by** at least one of:
a lower frequency bound which is about 0.9*(HR/60) Hz at all times, and
an upper frequency bound which is about 6 + 1.5*[(HR/60) - 1] Hz at all times,
wherein said HR is said heart rate in units of beats per minute.

14. The method (10, 20), apparatus (400) or system (120) of claim 2, 6 or 8, wherein said at least one CPX measure selected from the group consisting of ratio of ventilation efficiency to carbon dioxide production rate and oxygen uptake efficiency slope.

## Patentansprüche

1. Verfahren (10, 20) zum Schätzen der Belastungsfähigkeit einer Versuchsperson mittels Ausgangshochfrequenzsignalen (124), die zur Versuchsperson während einer körperlichen Belastung übertragen werden, und Eingangshochfrequenzsignalen (125), die von der Versuchsperson während einer körperlichen Belastung empfangen werden, wobei das Verfahren (10, 20) aufweist:
Verarbeiten des Eingangshochfrequenzsignals, wobei das Verarbeiten das Bestimmen (12) einer Phasenverschiebung der Eingangshochfrequenzsignale (125) relativ zu den Ausgangshochfrequenzsignale (124) aufweist, um ein verarbeitetes Eingangssignal bereitzustellen, das die Phasenverschiebung aufweist;
wobei das Verfahren (10, 20) ferner aufweist:
Filtern (34) des verarbeiteten Signals mittels eines dynamisch variablen Bandpassfilters, der durch ein Frequenzband gekennzeichnet ist, das als Reaktion auf eine Änderung eines physiologischen Zustands der Versuchsperson dynamisch angepasst wird, wodurch ein gefiltertes Signal bereitgestellt wird;
Berechnen (14) der Herzleistung beruhend auf der Phasenverschiebung; und
Verwenden (16) der Herzleistung zum Schätzen der Belastungsfähigkeit der Versuchsperson.

2. Verfahren (10, 20) nach Anspruch 1, das ferner das Beschaffen (40) mindestens eines CPX-Maßes, das eine kardiopulmonale Belastungsprüfung betrifft, und das Kombinieren der Herzleistung mit dem mindestens einen CPX-Maß aufweist, um die Belastungsfähigkeit der Versuchsperson zu schätzen.

3. Verfahren (10, 20) nach Anspruch 1, das ferner das Reduzieren oder Beseitigen einer (26) Amplitudenmodulation der Eingangshochfrequenzsignale (125) aufweist, um Eingangshochfrequenzsignale (125) mit einer im Wesentlichen konstanten Hüllkurve bereitzustellen.

4. Verfahren (10, 20) nach Anspruch 1, das ferner das Mischen der Ausgangshochfrequenzsignale (124) und der Eingangshochfrequenzsignale (125), um ein gemischtes Hochfrequenzsignal bereitzustellen, und das Ausfiltern eines Anteils des gemischten Hochfrequenzsignals aufweist, um ein Signal-Rausch-Verhältnis eines verbleibenden Anteils des gemischten Hochfrequenzsignals wesentlich zu erhöhen.

5. Vorrichtung (400) zum Schätzen der Belastungsfähigkeit einer Versuchsperson mittels Ausgangshochfrequenzsignalen (124), die zur Versuchsperson während einer körperlichen Belastung übertragen werden, und Eingangshochfrequenzsignalen (125), die von der Versuchsperson während einer körperlichen Belastung empfangen werden, wobei die Vorrichtung (400) aufweist:
eine Phasenverschiebungsbestimmungseinrichtung (50), die zum Bestimmen einer Phasenverschiebung der Eingangshochfrequenzsignale (125) relativ zu den Ausgangshochfrequenzsignale (124) konfiguriert ist, um ein verarbeitetes Signal bereitzustellen, das die Phasenverschiebung aufweist;
wobei die Vorrichtung (400) ferner aufweist:
ein Filtereinheit (46), die zum Filtern des verarbeiteten Signals mittels eines dynamisch variablen Bandpassfilter konfiguriert ist, der durch ein Frequenzband gekennzeichnet ist, das als Reaktion auf eine Änderung eines physiologischen Zustands der Versuchsperson dynamisch angepasst wird, um dadurch ein gefiltertes Signal bereitzustellen; und
einen Herzleistungsrechher (52), der zum Berechnen der Herzleistung beruhend auf der Phasenverschiebung konfiguriert ist; und
eine Belastungsfähigkeitsschätzungseinrichtung (54), die zum Verwenden der Herzleistung zum Schätzen der Belastungsfähigkeit der Versuchsperson konfiguriert ist.

6. Vorrichtung (400) nach Anspruch 5, wobei die Belastungsfähigkeitsschätzungseinrichtung (54) zum Kombinieren der Herzleistung mit mindestens einem CPX-Maß konfiguriert ist, um die Belastungsfähigkeit der Versuchsperson zu schätzen.

7. System (120) zum Schätzen der Belastungsfähigkeit einer Versuchsperson, das aufweist:
einen Hochfrequenzgenerator (122) zum Erzeugen von Ausgangshochfrequenzsignalen (124);
eine Vielzahl von Elektroden (125), die zum Übertragen der Ausgangshochfrequenzsignale (124) zur Versuchsperson und zum Abtasten der Eingangshochfrequenzsignale (125) von der Versuchsperson konfiguriert sind; und
die Vorrichtung (400) nach Anspruch 5.

8. System (120) nach Anspruch 7, das ferner ein kardiopulmonales Belastungsprüfsystem (150) aufweist, das konfiguriert ist, mindestens ein CPX-Maß bereitzustellen, wobei die Belastungsfähigkeitsschätzungseinrichtung (54) zum Kombinieren der Herzleistung mit mindestens einem CPX-Maß konfiguriert ist, um die Belastungsfähigkeit der Versuchsperson zu schätzen.

9. Vorrichtung (400) oder System (120) nach Anspruch 5 oder 7, wobei die Vorrichtung (400) ferner eine Hüllkurvenbeseitigungseinheit (135) aufweist, die zum Reduzieren oder Beseitigen einer Amplitudenmodulation der Eingangshochfrequenzsignale bestimmt und konfiguriert ist, um Eingangshochfrequenzsignale (125) mit einer im Wesentlichen konstanten Hüllkurve bereitzustellen.

10. Vorrichtung (400) oder System (120) nach Anspruch 5 oder 7, wobei die Vorrichtung (400) ferner aufweist:
einen Mischer (128), der zum Mischen der Ausgangshochfrequenzsignale (124) und der Eingangshochfrequenzsignale (125) konfiguriert ist, um ein gemischtes Hochfrequenzsignal bereitzustellen; und
einen Hochfrequenzfilter (132) zum Ausfiltern eines Anteils des gemischten Hochfrequenzsignals, um ein Signal-Rausch-Verhältnis eines verbleibenden Anteils des gemischten Hochfrequenzsignals wesentlich zu erhöhen

11. Verfahren (10, 20), Vorrichtung (400) oder System (120) nach Anspruch 1, 5 oder 7, wobei die Herzleistung mittels einer linearen Beziehung zwischen der Phasenverschiebung und der Herzleistung berechnet wird.

12. Verfahren (10, 20), Vorrichtung (400) oder System (120) nach Anspruch 1, 5 oder 7, wobei der physiologische Zustand eine Herzfrequenz der Versuchsperson ist.

13. Verfahren (10, 20), Vorrichtung (400) oder System (120) nach Anspruch 12, wobei der Filter **gekennzeichnet ist durch**:
eine untere Frequenzgrenze, die ständig etwa 0,9*(HR/60) Hz beträgt, und/oder eine obere Frequenzgrenze, die ständig etwa 6 + 1,5*[(HR/60) - 1] Hz beträgt,
wobei HR die Herzfrequenz in Einheiten von Schlägen pro Minute ist.

14. Verfahren (10, 20), Vorrichtung (400) oder System (120) nach Anspruch 2, 6 oder 8, wobei das mindestens eine CPX-Maß aus der Gruppe ausgewählt ist, die aus dem Verhältnis der Beatmungseffizienz zur Kohlendioxiderzeugungsrate und der Sauerstoffaufnahmesteigung besteht.

## Revendications

1. Procédé (10, 20) d'estimation de l'aptitude à l'exercice d'un sujet, recourant à des signaux radiofréquence de sortie (124) transmis au sujet pendant l'exercice et à des signaux radiofréquence d'entrée (125) reçus du sujet pendant l'exercice, ledit procédé (10, 20) comprenant :
le traitement du signal radiofréquence d'entrée, ledit traitement comprenant la détermination (12) d'un déphasage des signaux radiofréquence d'entrée (125) par rapport aux signaux radiofréquence de sortie (124), afin de délivrer un signal d'entrée traité comprenant le déphasage ;
ledit procédé (10, 20) comprenant en outre :
le filtrage (34) du signal traité en utilisant un filtre passe-bande dynamiquement variable **caractérisé par** une bande de fréquence dynamiquement adaptée en réaction à un changement d'un état physiologique du sujet, un signal filtré étant ainsi délivré ;
le calcul (14) du débit cardiaque sur la base du déphasage ; et
l'exploitation (16) du débit cardiaque afin d'estimer l'aptitude à l'exercice du sujet.

2. Procédé (10, 20) selon la revendication 1, comprenant en outre l'obtention (40) d'au moins une mesure CPX relevant d'un test d'exercice cardiopulmonaire, et la combinaison du débit cardiaque à la ou aux mesures CPX afin d'estimer l'aptitude à l'exercice du sujet.

3. Procédé (10, 20) selon la revendication 1, comprenant en outre la réduction ou la suppression (26) de la modulation d'amplitude des signaux radiofréquence d'entrée (125) de manière à délivrer des signaux radiofréquence d'entrée (125) d'enveloppe sensiblement constante.

4. Procédé (10, 20) selon la revendication 1, comprenant en outre le mélange des signaux radiofréquence de sortie (124) et des signaux radiofréquence d'entrée (125) de manière à délivrer un signal radiofréquence mélangé, et le filtrage d'une partie du signal radiofréquence mélangé de manière à augmenter sensiblement un rapport signal/bruit d'une partie restante du signal radiofréquence mélangé.

5. Appareil (400) permettant d'estimer l'aptitude à l'exercice d'un sujet, recourant à des signaux radiofréquence de sortie (124) transmis au sujet pendant l'exercice et à des signaux radiofréquence d'entrée (125) reçus du sujet pendant l'exercice, ledit appareil (400) comprenant :
une unité de détermination de déphasage (50) prévue pour déterminer un déphasage des signaux radiofréquence d'entrée (125) par rapport aux signaux radiofréquence de sortie (124), afin de délivrer un signal d'entrée traité comprenant le déphasage ;
ledit appareil (400) comprenant en outre :
une unité de filtrage (46) prévue pour filtrer le signal traité en utilisant un filtre passe-bande dynamiquement variable **caractérisé par** une bande de fréquence dynamiquement adaptée en réaction à un changement d'un état physiologique du sujet, un signal filtré étant ainsi délivré ; et
un calculateur de débit cardiaque (52) prévu pour calculer le débit cardiaque sur la base du déphasage ; et
une unité d'estimation d'aptitude à l'exercice (54) prévue pour exploiter le débit cardiaque afin d'estimer l'aptitude à l'exercice du sujet.

6. Appareil (400) selon la revendication 5, où l'unité d'estimation d'aptitude à l'exercice (54) est prévue pour combiner le débit cardiaque à au moins une mesure CPX afin d'estimer l'aptitude à l'exercice du sujet.

7. Système (120) permettant d'estimer l'aptitude à l'exercice d'un sujet, comprenant :
un générateur de radiofréquence (122) destiné à générer des signaux radiofréquence de sortie (124) ;
une pluralité d'électrodes (125) prévues pour transmettre les signaux radiofréquence de sortie (124) au sujet et pour détecter les signaux radiofréquence d'entrée (125) du sujet ; et
l'appareil (400) selon la revendication 5.

8. Système (120) selon la revendication 7, comprenant en outre un système de test d'exercice cardiopulmonaire (150) prévu pour délivrer au moins une mesure CPX, l'unité d'estimation de l'aptitude à l'exercice (54) étant prévue pour combiner le débit cardiaque à au moins une mesure CPX afin d'estimer l'aptitude à l'exercice du sujet.

9. Appareil (400) ou système (120) selon la revendication 5 ou la revendication 7, où ledit appareil (400) comprend en outre une unité de suppression d'enveloppe (135) prévue et configurée pour réduire ou supprimer la modulation d'amplitude des signaux radiofréquence d'entrée de manière à délivrer des signaux radiofréquence d'entrée (125) d'enveloppe sensiblement constante.

10. Appareil (400) ou système (120) selon la revendication 5 ou la revendication 7, où ledit appareil (400) comprend en outre :
une unité de mélange (128) prévue pour mélanger les signaux radiofréquence de sortie (124) et les signaux radiofréquence d'entrée (125) afin de délivrer un signal radiofréquence mélangé ; et
un filtre radiofréquence (132) destiné à filtrer une partie du signal radiofréquence mélangé de manière à augmenter sensiblement un rapport signal/bruit d'une partie restante du signal radiofréquence mélangé.

11. Procédé (10, 20), appareil (400) ou système (120) selon la revendication 1, 1a revendication 5 ou la revendication 7, où le débit cardiaque est calculé en exploitant une relation linéaire entre le déphasage et le débit cardiaque.

12. Procédé (10, 20), appareil (400) ou système (120) selon la revendication 1, la revendication 5 ou la revendication 7, où l'état physiologique est une fréquence cardiaque du sujet.

13. Procédé (10, 20), appareil (400) ou système (120) selon la revendication 12, où le filtre est **caractérisé par** au moins un des éléments suivants :
une limite inférieure de fréquence qui est toujours sensiblement égale à 0,9*(HR/60) Hz, et
une limite supérieure de fréquence qui est toujours sensiblement égale à 6 + 1,5*[(HR/60) - 1] Hz,
HR étant la fréquence cardiaque en unités de battements par minute.

14. Procédé (10, 20), appareil (400) ou système (120) selon la revendication 2, la revendication 6 ou la revendication 8, où la ou les mesures CPX sont sélectionnées dans le groupe comprenant le rapport entre l'efficacité de ventilation et le débit de production de dioxyde de carbone et la pente d'efficacité de consommation d'oxygène.
